# EUROPÄISCHE PATENTANMELDUNG

(11) **EP 3 628 226 A1**
(43) Veröffentlichungstag der Anmeldung: **01.04.2020**
(21) Anmeldenummer: 18197165.6
(22) Anmeldetag: 27.09.2018
(51) Int. Cl.: A61B 6/00, A61B 34/10

(54) **VERFAHREN ZUR ÜBERWACHUNG EINER GEWEBEENTNAHME MITTELS EINES RÖNTGEN-BILDGEBUNGSSYSTEMS**

(71) Anmelder: Siemens Healthcare GmbH, 91052 Erlangen (DE)
(72) Erfinder: Mertelmeier, Thomas, 91058 Erlangen (DE); Biniazan, Ramyar, 90402 Nürnberg (DE)

(57) **Zusammenfassung**

Die Erfindung betrifft ein Verfahren zur Überwachung einer Gewebeentnahme mittels eines Röntgen-Bildgebungssystems (2), wobei die Gewebe-Entnahme mittels eines Entnahme-Instruments (17) aus einer Läsion (L) in einem zu untersuchenden Brustgewebe (BG) erfolgt. Das Verfahren umfasst die folgenden Schritte:
- Erfassen (S1) von ersten durch ein Kontrastmittel beeinflussten Röntgenprojektionsmessdaten (RMD) des in einem Bildfeld des Röntgen-Bildgebungssystems befindlichen Brustgewebes, wobei die kontrastmittelbeeinflussten ersten Röntgenprojektionsmessdaten mit Röntgenphotonen erzeugt werden, die eine Photonenenergie oberhalb einer Absorptionskante des eingesetzten Kontrastmittels aufweisen,
- Rekonstruieren (S2) eines ersten Bilddatensatzes (BD1) auf Basis der erfassten Röntgenprojektionsmessdaten,
- Bestimmen (S3) anhand des rekonstruierten ersten Bilddatensatzes einer aktuellen Position (APOS) der Läsion in Bezug zu einem Referenz-Koordinatensystem.

## Beschreibung

Die Erfindung betrifft ein Verfahren zur Überwachung einer Gewebeentnahme aus Brustgewebe mittels eines Röntgen-Bildgebungssystems. Dabei werden durch ein Kontrastmittel beeinflusste Bilddaten unter Einsatz ausschließlich hochenergetischer Röntgenstrahlung erzeugt.

Für die Früherkennung von Mammakarzinomen spielt die klassische Mammographie eine wichtige Rolle. Bei der klassischen Mammographie wird von einer weiblichen oder männlichen Brust eine Durchleuchtungs-Röntgenaufnahme erstellt. Die dabei eingesetzte Röntgenstrahlung entspricht weicher Strahlung mit einer Energie von etwa 25 bis 35 keV. Zur Detektion der Röntgenstrahlung kommen direkte oder indirekte digitale Detektoren zum Einsatz, um die emittierte Röntgenstrahlung zu erfassen. Direkte digitale Detektoren wandeln Röntgenstrahlung direkt in ein elektrisches Signal um. Indirekte digitale Detektoren dagegen wandeln die Röntgenstrahlung erst in sichtbares Licht, das anschließend in ein elektrisches Signal umgewandelt wird. Die Röntgenaufnahmen werden auf einer speziellen Mammographie-Befundstation betrachtet, welche ein oder zwei Graustufenmonitore umfasst, mit denen die Röntgenbilder bildlich dargestellt werden.

Nachteilig ist, dass die Sensitivität der klassischen Mammografie mit zunehmender Brustdichte abnimmt. Mit anderen Worten werden Läsionen, insbesondere Tumoren mit zunehmender Gewebedichte schlechter erkannt. Zudem gibt es bestimmte Tumortypen, die mittels Röntgenmammografie grundsätzlich schwer zu erkennen sind. Zudem besteht bei der klassischen ,Durchleuchtungsmammographie' das Problem, dass Läsionen durch andere Gewebestrukturen verdeckt werden und somit schlecht identifiziert bzw. lokalisiert werden können.

Um diese Nachteile zu überwinden, ist bekannt, nach einer intravenösen Verabreichung von Kontrastmittel wie bspw. Jod eine kontrastverstärkte Zweispektren-Mammographie oder (CEDEM = contrast enhanced dual energy mammography = kontrastverstärkte dual-energetische Mammographie) durchzuführen. Unter Beibehaltung einer Brustkompression wird dabei eine Hochenergieaufnahme gefolgt von einer Niedrigenergieaufnahme durchgeführt oder umgekehrt. Anschließend erfolgt durch eine Registrierung und eine gewichtete Subtraktion eine Erstellung eines rekombinierten Ergebnisbildes, in dem im Wesentlichen die Bereiche besonders gut sichtbar dargestellt werden, in denen sich das Kontrastmittel angereichert hat. Insbesondere Karzinome zeichnen sich dadurch aus, aufgrund ihrer verstärkten Vaskularisierung (Neoangiogenese) und einer erhöhten Gefäßpermeabilität verstärkt Kontrastmittel anzureichern. Mit der Zweispektren-Mammografie kann so der Jodkontrast maximiert und der sonst okkulte Tumor sichtbar gemacht werden. Die Röntgenstrahlenenergien für die Hochenergie- und die Niedrigenergieaufnahmen werden typischerweise derart gewählt, dass der Wert der niedrigeren Energie in der Nähe des Energiewerts der (Röntgen-)Absorptionskante des verwendeten Kontrastmittels für Röntgenstrahlung liegt und der Wert der höheren Energie weit oberhalb des Energiewerts dieser Absorptionskante. Beispielsweise liegt die K-Absorptionskante für Röntgenstrahlung des Kontrastmittels Jod, im Folgenden auch kurz Röntgenabsorptionskante genannt, bei 33,17keV. Auch andere Kontrastmittel sind denkbar, z.B. Silber enthaltende Substanzen.

Die 3D-Brust-Tomosynthese bietet ein Bildgebungsverfahren, bei dem die Brust aus vielen unterschiedlichen Winkeln aufgenommen wird. Beispielsweise werden Aufnahmen in Winkeln von 15 bis 50 Grad aufgenommen. Insgesamt werden zum Beispiel zwischen 9 und 25 Aufnahmen aus unterschiedlichen Winkeln mit niedriger Dosis aufgenommen, so dass die Gesamtdosis in etwa der einer klassischen zweidimensionalen Mammographieaufnahme entspricht. Aus den erfassten Projektionsdaten werden Bilder für einzelne Schichten des Brustgewebes errechnet, insgesamt kann ein dreidimensionaler Bilddatensatz entstehen, der einer Computertomographieaufnahme angenähert ist. Für die Rekonstruktion einer dreidimensionalen Abbildung eines zu untersuchenden Bereichs aus den erfassten Projektionsmessdaten kommt beispielsweise das Verfahren der gefilterten Rückprojektion zum Einsatz. Das resultierende dreidimensionale Bild kann zu Diagnosezwecken schichtweise oder zusammenhängend betrachtet werden. Da Schichten über und unter der jeweils zur Ansicht ausgewählten Schicht bei der Befundung ausgeblendet werden können, sind Gewebeveränderungen leichter zu erkennen.

Auch die Berechnung eines rekombinierten Dual-Energy-Mammogramms aus der 3D-Brust-Tomosynthese (CEDET) ist bekannt. Bei dieser Methode werden eine Hochenergie-Tomosynthese-Aufnahme und eine Niedrig-Energie-Tomosynthese-Aufnahme erstellt und ein Differenzbild aus den beiden Aufnahmen ermittelt.

Um die Bösartigkeit eines radiologisch erkannten Tumors abzuklären, kann mit dem Ziel einer labor-diagnostischen Befundung eine Biopsie durchgeführt werden. Dies geschieht typischerweise zu einem späteren Zeitpunkt losgelöst von der radiologischen Untersuchung. Eine konventionelle Biopsie, die unter Anwendung von weicher, also niederenergetischen Röntgenstrahlung unter Röntgenbildgebung durchgeführt wird, birgt erneut das Risiko, dass die zu biopsierende Läsion nicht erkannt wird und nicht präzise biopsiert werden kann. Eine Biopsie bei gleichzeitiger Aufnahme von Dual-Energie-Aufnahmen mit Kontrastmittel erhöht das medizinische Risiko für den Patienten wegen der erhöhten Strahlendosis.

Es besteht also die Aufgabe, Mittel bereit zu stellen, die es erlauben, bei geringer Strahlenbelastung für einen Patienten eine Läsion für eine bzw. während einer Biopsie zuverlässig darzustellen bzw. zu lokalisieren. Es besteht ferner die Aufgabe, diese Position über den Verlauf einer Biopsie hinweg zu überwachen.

Diese Aufgabe wird gelöst durch ein Verfahren zur Überwachung einer Gewebeentnahme aus Brustgewebe mittels eines Röntgen-Bildgebungssystems, entsprechende Recheneinheit, entsprechendes Röntgen-Bildgebungssystem, entsprechendes Computerprogrammprodukt und entsprechendes computerlesbares Medium gemäß den unabhängigen Ansprüchen. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Die vorliegende Erfindung betrifft in einem ersten Aspekt ein Verfahren zur Überwachung einer Gewebeentnahme mittels eines Röntgen-Bildgebungssystems, wobei die Gewebeentnahme mittels eines Entnahme-Instruments aus einer Läsion in einem zu untersuchenden Brustgewebe erfolgt. Das Verfahren umfasst eine Vielzahl von Schritten.

Eine Gewebeentnahme entspricht einer Biopsie, bei der ein Teil eines Gewebes, bspw. Tumorgewebe, aus einem Körper eines Untersuchungsobjektes entfernt wird. Dazu kann ein Entnahme-Instrument bspw. eine Biopsie-Nadel verwendet werden. Es sei explizit darauf hingewiesen, dass der Schritt der Gewebeentnahme selbst nicht von dem erfindungsgemäßen Verfahren umfasst ist. Vielmehr ist das erfindungsgemäße Verfahren ausgebildet, zeitgleich bzw. parallel zu einer Gewebeentnahme durchgeführt zu werden. Es kann auch vorgesehen sein, dass einzelne Schritte des erfindungsgemäßen Verfahrens mit Schritten der Gewebeentnahme verschachtelt ausgeführt werden. Dennoch sind das erfindungsgemäße Verfahren und die Gewebeentnahme selbst als getrennt voneinander zu betrachten.

Brustgewebe kann weibliches oder männliches Brustgewebe umfassen.

Ein erster Schritt betrifft ein Erfassen von ersten durch ein Kontrastmittel beeinflussten bzw. kontrastmittelbeeinflussten bzw. kontrastmittelgestützten Röntgenprojektionsmessdaten des in einem Bildfeld des Röntgen-Bildgebungssystems befindlichen Brustgewebes. Mit anderen Worten wird das zu untersuchende Brustgewebe umfassend eine Läsion mit Röntgenphotonen bestrahlt. Die Röntgenabschwächung gemäß der Gewebeverteilung in dem Brustgewebe und der Bestrahlungsrichtung wird mittels der ersten Röntgenprojektionsmessdaten erfasst.

Als durch ein Kontrastmittel beeinflusst bzw. kontrastmittelunterstützt sollen Projektionsmessdaten verstanden werden, die nach Gabe eines Kontrastmittels und dessen Transport in den Untersuchungsbereich, also das zu untersuchende Brustgewebe, aufgenommen wurden. Bevorzugt kommt Jod als Kontrastmittel zum Einsatz. Andere Kontrastmittel sind ebenfalls möglich und im Sinne der Erfindung. Die Wahl eines geeigneten Kontrastmittels kann zum Beispiel von der Art einer Läsion, Unverträglichkeiten und/oder anatomischen Besonderheiten des Patienten oder dergleichen abhängen. Bei Wahl eines anderen Kontrastmittels kann es zweckmäßig oder erforderlich sein, auch eingesetzte Filtermaterialien bzw. das Röntgenröhrenspektrum anzupassen.

Ein weiterer Schritt betrifft ein Rekonstruieren eines ersten Bilddatensatzes auf Basis der erfassten Röntgenprojektionsmessdaten. In diesem Schritt werden die ersten Röntgenprojektionsmessdaten in ein digital darstellbares Bild in Form des ersten Bilddatensatzes überführt.

Unter einer Bildrekonstruktion soll in diesem Zusammenhang ein Ermitteln einer Bilddarstellung auf der Basis der erfassten Messdaten verstanden werden. Bei erfassten zweidimensionalen Messdaten kann dies zum Beispiel eine einfache Aufbereitung, insbesondere eine Entstörung oder Korrektur der vorhandenen Messdaten, welche bereits eine Bilddarstellung enthalten, bedeuten. Eine Bildrekonstruktion kann jedoch bei einer Aufnahme von Projektionsmessdaten aus verschiedenen Richtungen auch die Rekonstruktion einer dreidimensionalen Abbildung umfassen.

Ein weiterer Schritt betrifft ein Bestimmen anhand des rekonstruierten ersten Bilddatensatzes einer aktuellen Position der Läsion in Bezug zu einem Referenz-Koordinatensystem.

Das Referenz-Koordinatensystem ist als das Bezugskoordinatensystem des Röntgen-Bildgebungssystems zu verstehen, mit dem die Röntgenprojektionsmessdaten erfasst werden. Es kann auch dem Koordinatensystem entsprechen, in welchem sich das Entnahme-Instrument bewegt. Die Bestimmung der aktuellen Position der Läsion kann einerseits manuell durch einen Benutzer anhand des ersten Bilddatensatzes erfolgen, bspw. über eine Markierung wenigstens einer Position mittels Maus-Cursor-Interaktion. Alternativ oder zusätzlich, bspw. mittels eines semi-automatischen Verfahrens, kann die Läsion mittels eines Bildanalyse-Verfahrens automatisch lokalisiert werden. Hierzu können bspw. Pixelwerte einzelner Pixel mit einem Schwellwert verglichen und dementsprechend der Läsion oder umliegendem Gewebe zugeordnet werden. Auch möglich ist die Anwendung von Algorithmen zur Bestimmung von Randpixeln der Läsion. Das Bestimmen der aktuellen Position der Läsion kann auch ein Bestimmen der Größe, des Volumens und/oder der Ausdehnung der Läsion in wenigstens einer Raumdimension umfassen.

Das erfindungsgemäße Verfahren ist dadurch gekennzeichnet, dass die kontrastmittelbeeinflussten ersten Röntgenprojektionsmessdaten mit Röntgenphotonen erzeugt werden, die eine Photonenenergie oberhalb einer Absorptionskante des eingesetzten Kontrastmittels aufweisen.

Die (Röntgen-)Photonenenergie oder auch Röntgenstrahlenergie beschreibt in der Regel die mittlere Energie des Röntgenquantenenergiespektrums der emittierten Röntgenphotonen. Beispielsweise beträgt bei einer Röntgenröhrenspannung einer Röntgenstrahlenquelle für eine Mammographieaufnahme von 30kV die mittlere Energie des Röntgenstrahlenspektrums etwa 20keV. Als Röntgenenergiespektrum soll die Energieverteilung der von einer Röntgenröhre erzeugten und ausgesandten Röntgenquanten verstanden werden. Üblicherweise weisen von einer Röntgenstrahlenquelle emittierte Röntgenstrahlen nicht nur eine diskrete Energie, sondern ein ganzes Spektrum unterschiedlicher Energiewerte auf. Dabei lässt sich zusätzlich zu dem genannten Mittelwert der Energieverteilung der emittierten Röntgenstrahlung ein Energiewert e * U zuweisen, der eine minimale Wellenlänge bzw. Maximalenergie der Röntgenquanten der Röntgenstrahlung festlegt (Dieser Wert würde in dem konkreten Beispiel 30keV betragen und soll an dieser Stelle nur zur besseren Unterscheidung erwähnt werden). In diesem Sinne sind die Begriffe "hoch" und "niedrig" in Bezug zu Röntgenstrahlen mit hoher Energie und niedriger Energie relativ zu der Energie der Röntgenabsorptionskante des eingesetzten Kontrastmittels zu verstehen.

Die erfindungsgemäße Vorgehensweise basiert auf der Erkenntnis, dass ein guter bzw. ausreichender Bildkontrast zwischen einer Läsion, insbesondere einem Tumor und umliegendem Mamma-Gewebe mittels hochenergetischer Röntgenphotonen erreicht werden kann. Mit anderen Worten erzielen Röntgenphotonen, die eine Photonenenergie oberhalb der Röntgenabsorptionskante, auch K-Kante genannt, einen Bildkontrast, der für eine Anwendung für eine Biopsie bzw. Gewebeentnahme ausreicht. Mit anderen Worten basiert der erste Bilddatensatz ausschließlich auf hochenergetischen Röntgenprojektionsmessdaten, wobei die Röntgenphotonen eine Photonenenergie oberhalb der spezifischen Röntgenabsorptionskante des verwendeten Kontrastmittels aufweisen. Diese Vorgehensweise verzichtet vorteilhaft auf das Erfassen von niederenergetischen Röntgenprojektionsmessdaten und die damit verbundene höhere Strahlendosis für den Patienten und ist dennoch geeignet, eine Läsion klar abzubilden bzw. zu lokalisieren.

Ein anderer Aspekt der vorliegenden Erfindung betrifft eine Recheneinheit zur Überwachung einer Gewebeentnahme mittels eines Röntgen-Bildgebungssystems, wobei die Gewebe-Entnahme mittels eines Entnahme-Instruments aus einer Läsion in einem zu untersuchenden Brustgewebe erfolgt, aufweisend Mittel zum Durchführen des erfindungsgemäßen Verfahrens. Vorteilhaft ist die Recheneinheit in das Röntgen-Bildgebungssystem integriert. Alternativ kann die Recheneinheit auch entfernt bzw. abgelegen angeordnet sein. Die Recheneinheit kann ausgebildet sein, insbesondere den Schritt des Bestimmens der aktuellen Position der Läsion, aber auch das gesamte erfindungsgemäße Verfahren, für das Röntgen-Bildgebungssystem oder für eine Vielzahl von Anlagen durchzuführen, z.B. in einem Radiologie-Zentrum oder Krankenhaus umfassend mehrere Magnetresonanz-Anlagen.

Ein anderer Aspekt der vorliegenden Erfindung betrifft ein Röntgen-Bildgebungssystem zur Überwachung einer Gewebeentnahme, wobei die Gewebe-Entnahme mittels eines Entnahme-Instruments aus einer Läsion in einem zu untersuchenden Brustgewebe erfolgt. Das Röntgen-Bildgebungssystem umfasst
- eine Röntgenstrahlenquelle zum Emittieren von Röntgenphotonen,
- eine Erfassungseinheit zum Erfassen von ersten durch ein Kontrastmittel beeinflussten Röntgenprojektionsmessdaten des in einem Bildfeld des Röntgen-Bildgebungssystems befindlichen Brustgewebes,
- eine Rekonstruktionseinheit zum Rekonstruieren eines ersten Bilddatensatzes auf Basis der erfassten Röntgenprojektionsmessdaten,
- eine Bestimmungseinheit zum Bestimmen einer aktuellen Position der Läsion anhand des rekonstruierten ersten Bilddatensatzes in Bezug zu einem Referenz-Koordinatensystem.
Die Röntgenstrahlenquelle ist ausgebildet, Röntgenphotonen zu emittieren, die eine Photonenenergie oberhalb einer Absorptionskante des eingesetzten Kontrastmittels aufweisen. Diese Röntgenphotonen werden zur Erzeugung der ersten Röntgenprojektionsmessdaten eingesetzt.

Das Röntgen-Bildgebungssystem kann vorteilhaft auch eine Ermittlungseinheit zum Ermitteln einer Entnahme-Trajektorie für das Entnahme-Instrument, die die bestimmte Position der Läsion schneidet, umfassen.

Die Erfassungseinheit kann als Röntgenstrahlendetektor, direkt oder indirekt konvertierend, ausgebildet sein. Die Erfassungseinheit kann auch als Schnittstelle einer Recheneinheit des Röntgen-Bildgebungssystems zur Weiterverarbeitung der Röntgenprojektionsmessdaten ausgebildet sein, über welche Schnittstelle die Messdaten an die Recheneinheit übertragen werden.

Erfassungseinheit, Rekonstruktionseinheit, Bestimmungseinheit und Ermittlungseinheit können von der Recheneinheit des Röntgen-Bildgebungssystems umfasst sein. Sie können jeweils als einzelne oder mehrere Komponenten, also Prozessor oder Computer ausgebildet sein, die zudem räumlich zusammenhängend, aber auch räumlich getrennt voneinander angeordnet sein können.

Das Röntgen-Bildgebungssystem kann vorteilhaft auch das Entnahme-Instrument umfassen. Es kann ferner auch eine AnzeigeVorrichtung umfassen, auf welcher für einen Betrachter ein rekonstruierter Bilddatensatz, bspw. der erste Bilddatensatz angezeigt werden kann. Besonders bevorzugt ist das Röntgen-Bildgebungssystem als Mammographie-System ausgebildet.

Ein weiterer Aspekt der vorliegenden Erfindung betrifft ein Computerprogrammprodukt mit einem Computerprogramm, welches direkt in eine Speichereinheit eines Röntgen-Bildgebungssystems ladbar ist, mit Programmabschnitten, um die Schritte des erfindungsgemäßen Verfahrens auszuführen, wenn das Computerprogramm in dem Röntgen-Bildgebungssystem ausgeführt wird.

Ein weiterer Aspekt der vorliegenden Erfindung betrifft ein computerlesbares Medium, auf welchem von einer Recheneinheit des Röntgen-Bildgebungssystems einlesbare und ausführbare Programmabschnitte gespeichert sind, um alle Schritte des erfindungsgemäßen Verfahrens auszuführen, wenn die Programmabschnitte von der Recheneinheit des Röntgen-Bildgebungssystems ausgeführt werden. Vorteilhaft kann insbesondere das Bestimmen der aktuellen Position der Läsion oder das Bestimmen der Entnahme-Trajektorie auf einem Computer, beispielsweise in der Recheneinheit des Röntgen-Bildgebungssystems, ausgeführt werden.

In einer bevorzugten Ausführungsform der Erfindung erfolgt ein Ermitteln einer Entnahme-Trajektorie für das Entnahme-Instrument, die die bestimmte aktuelle Position der Läsion umfasst. Dieser Schritt berücksichtigt eine Ausgangsposition des Entnahme-Instruments sowie die aktuelle Position der Läsion. Die Entnahme-Trajektorie beschreibt einen Bewegungspfad für das Entnahme-Instrument, auf dem das Entnahme-Instrument die Läsion wenigstens einmal schneidet, trifft oder kreuzt, um dort Gewebe auszulösen. Bevorzugt ist die Entnahme-Trajektorie geradlinig ausgebildet. Das Ermitteln der Entnahme-Trajektorie kann automatisch durch eine Recheneinheit, aber auch manuell durch einen Benutzer erfolgen.

In einer bevorzugten Ausführungsform der Erfindung werden die Röntgenphotonen von einer Röntgenstrahlenquelle des Röntgen-Bildgebungssystems emittiert, an welcher eine Spannung in einem Bereich von 40kV bis 49kV anliegt. Röntgenröhrenspannungen innerhalb dieses Bereichs verursachen jeweils ein Röntgenphotonenenergiespektrum hervor, dessen mittlere Energie oberhalb, bevorzugt weit oberhalb der Röntgenabsorptionskante des Kontrastmittels Jod liegt. Spannungswerte innerhalb dieses Bereichs verursachen eine mittlere Photonenenergie im Bereich von etwa 30keV bis 40keV. Dies hängt zusätzlich von der Wahl bzw. Ausgestaltung des Röntgenstrahlenfilters ab. Hier ist typischerweise ein Titan- oder Kupfer-Filter vorgesehen. Ein Titan-Filter hätte in dieser Ausgestaltung beispielsweise eine Dicke von 0,4mm bis 1,4mm, ein Kupfer-Filter wäre dünner. Entsprechend einer besonders bevorzugten Ausführungsform der Erfindung liegt die Röntgenröhrenspannung im Bereich von 45kV bis 49kV an. Spannungswerte innerhalb dieses Bereichs verursachen eine mittlere Photonenenergie von etwa 35keV bis 40keV. Tests haben ergeben, dass diese mittlere Photonenenergie einen besonders guten Kontrast zwischen Läsion und umliegendem Brustgewebe in dem ersten Bilddatensatz bewirkt.

In einer anderen vorteilhaften Ausgestaltung der vorliegenden Erfindung umfasst der erste Bilddatensatz wenigstens zwei zweidimensionale oder einen dreidimensionalen Bilddatensatz. Mit anderen Worten kann der erste Bilddatensatz als kontrastmittelgestützte Stereo-Mammographie(aufnähme) ausgebildet sein. In diesem Fall werden Projektionsmessdaten aus zwei leicht zueinander versetzten Blickrichtungen auf das Brustgewebe erfasst und unabhängig voneinander zu zwei-dimensionalen Einzelbildern rekonstruiert. Aus diesen kann dann beispielsweise durch Triangulation die Tiefeninformation extrahiert werden. Alternativ dazu kann die Projektionsmessdatenerfassung die kontrastmittelgestützte Aufnahme von Projektionen in einer Vielzahl von verschiedenen Blickrichtungen umfassen, die gemeinsam zu einem dreidimensionalen Tomosynthese-Bilddatensatz rekonstruiert werden. Hier ist die Tiefeninformation dann über die Tiefenkoordinate des die Läsion umfassenden Schichtbildes unmittelbar enthalten. Die Wahl der Ausbildung des ersten Bilddatensatzes kann insbesondere von den Kapazitäten des verwendeten Röntgen-Bildgebungssystems abhängen, alternativ oder zusätzlich kann die Art des zu untersuchenden Brustgewebes bzw. der Läsion eine Stereo-Mammographie oder eine Tomosynthese erfordern.

In einer weiteren vorteilhaften Ausführung der vorliegenden Erfindung werden vor Erfassen der ersten Röntgenprojektionsmessdaten durch das Kontrastmittel beeinflusste Übersichtsprojektionsmessdaten erfasst werden. Die Übersichtsprojektionsmessdaten decken bevorzugt ein größeres Bildfeld ab als die ersten Röntgenprojektionsmessdaten. Das Bildfeld ist als Field of View (FoV) oder Blickfeld des Röntgen-Bildgebungssystems zu verstehen. Die Übersichtprojektionsmessdaten dienen einer Identifizierung und ersten, groben Lokalisierung der Läsion in dem Brustgewebe und ggf. auch einer Ausrichtung bzw. Anpassung des Bildfeldes des Röntgen-Bildgebungssystems auf die Läsion. Mit anderen Worten kann vorgesehen sein, dass das Untersuchungsobjekt basierend auf einer aus den Übersichtsprojektionsmessdaten rekonstruierten Übersichtsaufnahme neu positioniert wird, um im Anschluss mit den ersten Projektionsmessdaten die Läsion tatsächlich zu erfassen. Das Blickfeld wird für die Erfassung der ersten Röntgenprojektionsmessdaten bevorzugt entsprechend der Größe der Läsion verkleinert, sodass die Läsion mit dem ersten Bilddatensatz bestmöglich abgebildet werden kann.

In einer weiteren vorteilhaften Ausführungsform der vorliegenden Erfindung wird die aktuelle Position der Läsion in dem ersten Bilddatensatz vor dem Ermitteln der Entnahme-Trajektorie mit einer Referenzposition der Läsion in einem Referenz-Bilddatensatz registriert. Typischerweise liegt zum Zeitpunkt einer Gewebe-Entnahme und der Durchführung des erfindungsgemäßen Verfahrens bereits ein radiologischer Befund in Bezug auf die Läsion in dem zu untersuchenden Brustgewebe vor. Mit anderen Worten ist beispielsweise wenigstens eine Zweispektren-Mammographie bzw. eine Tomosynthese-Aufnahme des Brustgewebes umfassend die Läsion vorhanden, die jeweils als Referenz-Bilddatensatz fungieren können. Es ist also erfindungsgemäß vorgesehen, die Läsion in dem ersten Bilddatensatz mit der Referenz-Position der Läsion in dem Referenz-Bilddatensatz entsprechend dem radiologischen Befund zu registrieren. Mit anderen Worten bewirkt dieser Schritt eine Abbildung der Läsionen in den beiden Bilddatensätzen aufeinander. Dazu kann ein beliebiges, an sich bekanntes und geeignetes Registrierungsverfahren eingesetzt werden. Beispielsweise eignen sich in der Mammographie besonders gut folgende Verfahren: elastische bzw. deformierbare (non-rigid) Registrierung, Multi-Resolution Registration, Feature Based Registration oder dergleichen. Bei der Registrierung werden zumindest Bildinformationen in Bezug auf die Läsion berücksichtigt. Durch die Registrierung des ersten Bilddatensatzes mit dem Referenz-Bilddatensatz kann die Bestimmung der Position der Läsion in dem ersten Bilddatensatz optimiert und/oder bestätigt werden, da derart Informationen in Bezug auf die Position der Läsion des Referenz-Bilddatensatzes, die im ersten Bilddatensatz fehlen können, für die Positionsbestimmung berücksichtigt werden können.

Gemäß einer besonders bevorzugten Ausführung wird für die Registrierung die Position wenigstens einer anatomischen Landmarke in dem ersten Bilddatensatz und dem Referenz-Bilddatensatz berücksichtigt. Da eine Voraufnahme, also ein Referenz-Bilddatensatz unter einer (leicht) anderen Kompression des Brustgewebes durchgeführt worden sein kann, ist eine Registrierung unter Berücksichtigung von zusätzlichen anatomischen Landmarken, die in dem ersten Bilddatensatz umfasst sind, besonders nützlich, um die Registrierungsgenauigkeit zu erhöhen und damit die Positionsbestimmung weiter zu verbessern. Anatomische Landmarken können bei der Mammographie insbesondere die Hautlinie des Brustgewebes, die Mamilla, größere Blutgefäße und/oder Mikrocalc oder dergleichen sein. Mit anderen Worten ist in dieser Ausführung vorgesehen, dass neben Bildinformation bezüglich der Läsion auch Bildinformation bezüglich wenigsten einer anatomischen Landmarke, bevorzugt mehrerer anatomischer Landmarken für eine Überlagerung des ersten Bilddatensatzes mit dem Referenz-Bilddatensatz berücksichtigt wird.

In einer weiteren vorteilhaften Ausgestaltung der Erfindung werden wenigstens zu einem Zeitpunkt zweite durch das Kontrastmittel beeinflusste Röntgenprojektionsmessdaten erfasst und zu einem zweiten Bilddatensatz rekonstruiert, während das Entnahme-Instrument eine Bewegung entlang der Entnahme-Trajektorie vollzieht. Mit anderen Worten wird wenigstens einmal, bevorzugt mehrfach zu verschiedenen Zeitpunkten, nach dem Bestimmen der Entnahme-Trajektorie basierend auf der Position der Läsion ein zweiter Bilddatensatz rekonstruiert. Diese Vorgehensweise dient der Überwachung der Bewegung des Entnahme-Instruments entlang der Entnahme-Trajektorie. Sollten sich während der Bewegung unerwartet Abweichungen, bspw. durch eine unvorhersehbare Patientenbewegung, ergeben, kann derart die Bewegung des Entnahme-Instruments vorteilhaft unterbrochen werden, und so bspw. eine Patientenverletzung vermieden werden.

In einer weiteren Ausführungsvariante der Erfindung werden dritte durch das Kontrastmittel beeinflusste Röntgenprojektionsdaten erfasst und zu einem dritten Bilddatensatz rekonstruiert, nachdem das Entnahme-Instrument die Bewegung entlang der Entnahme-Trajektorie vollzogen hat. Diese Vorgehensweise dient der Überprüfung des Brustgewebes bzw. der Läsion und ermöglicht, die Kontrolle, dass tatsächlich eine Gewebeprobe aus der Läsion entnommen wurde bzw. dass keine Verletzungen durch das Entnahme-Instrument verursacht wurden.

Besonders vorteilhaft wird der erste, der zweite und/oder der dritte Bilddatensatz instantan einem Benutzer angezeigt. Bevorzugt werden alle drei Bilddatensätze dem Benutzer zur Anzeige gebracht. Derart kann während des gesamten Biopsie-Verlaufs eine optische Kontrolle gewährleistet werden. Instantan umfasst in dieser Ausführung eine im Wesentlichen zeitlich unverzögerte bzw. kaum verzögerte Bild-Darstellung, sodass sofern erforderlich, eine schnelle Reaktion, bspw. Unterbrechung der Bewegung des Entnahme-Instruments, bewirkt werden kann. Diese Ausführung umfasst selbstredend auch eine instantane bzw. quasi-instantane Bild-Rekonstruktion.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten, Einheiten oder Schritte mit identischen Bezugszeichen versehen. Die Figuren sind in der Regel nicht maßstäblich. Es zeigen:
- FIG 1: eine Ansicht eines Röntgen-Bildgebungssystems in Form einer Mammographie-Anlage umfassend eine Recheneinheit, jeweils gemäß einer Ausführungsform der vorliegenden Erfindung,
- FIG 2: eine schematische Darstellung eines erfindungsgemäßen Verfahrens gemäß einem Ausführungsbeispiel der vorliegenden Erfindung,
- FIG 3: einen ersten Bilddatensatz gemäß dem Stand der Technik sowie einer Ausführungsform der vorliegenden Erfindung.

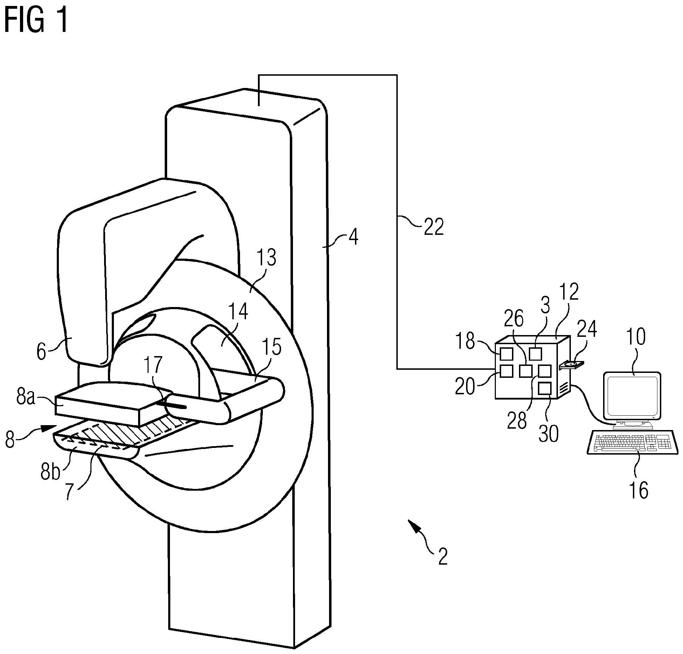

**Figur 1** zeigt eine Ansicht eines Röntgen-Bildgebungssystems in Form einer Mammographie-Anlage 2 umfassend eine Recheneinheit 12, jeweils gemäß einer Ausführungsform der vorliegenden Erfindung. Die erfindungsgemäße Mammographie-Anlage 2 weist ein Gehäuse 13 mit zumindest einer Öffnung 14 auf. Das Gehäuse 13 ist mit einem Hauptkörper 4 der Anlage 2 verbunden. Auf der dem Hauptkörper 4 abgewandten Seite des Gehäuses 13 ist eine Kompressionseinheit 8 aus Kompressionselement 8a und Objekttisch 8b so angeordnet, dass mit einer Röntgenstrahlenquelle 6 ein in der Kompressionseinheit 8 befindliches Objekt bspw. in Form eines Brustgewebes durchleuchtet werden kann. Die Röntgenstrahlung wird mit einer im Bereich des Objekttisches 8b befindlichen Erfassungseinheit in Form eines Röntgen-Strahlendetektors 7 aufgenommen. Die hier gezeigte Mammographie-Anlage 2 umfasst auch eine Biopsieeinheit 15. Diese ragt aus einer Öffnung 14 des Gehäuses 13 teilweise hervor. Die Öffnung 14 kann größer als gezeigt sein, so dass sie z.B. eine Schwenkbewegung von ±90° um eine Achse erlaubt, oder im Gehäuse 13 verschiebbar sein, um eine entsprechende Schwenkbewegung zu erlauben. Alternativ kann die Biopsieeinheit 15 auch mit vertikaler Orientierung angeordnet sein. Die Entnahmeeinheit 15 ist teilweise außerhalb des Gehäuses 13 angeordnet und umfasst an ihrem beweglichen Ende ein Entnahme-Instrument in Form einer Biopsie-Nadel 17.

Die Mammographie-Anlage 2 verfügt über eine Recheneinheit in Form eines Computersystems 12, welches als Computer ausgebildet ist und mit einer Anzeigeeinheit 10, beispielsweise zur graphischen Anzeige von rekonstruierten Mammographie-Bilddaten BD1, BD2, BD3 sowie einer Eingabeeinheit 16 verbunden ist. Bei der Anzeigeeinheit 10 kann es sich beispielsweise um einen LCD-, Plasma- oder OLED-Bildschirm handeln. Es kann sich weiterhin um einen berührungsempfindlichen Bildschirm handeln, welcher auch als Eingabeeinheit 16 ausgebildet ist. Ein solcher berührungsempfindlicher Bildschirm kann in das bildgebende Gerät integriert oder als Teil eines oder selbst als mobiles Gerät ausgebildet sein. Bei der Eingabeeinheit 10 handelt es sich beispielsweise um eine Tastatur, eine Maus, einen sogenannten "Touch-Screen" oder auch um ein Mikrofon zur Spracheingabe. Die Eingabeeinheit 16 kann auch eingerichtet sein, um Bewegungen eines Benutzers zu erkennen und in entsprechende Befehle zu übersetzen.

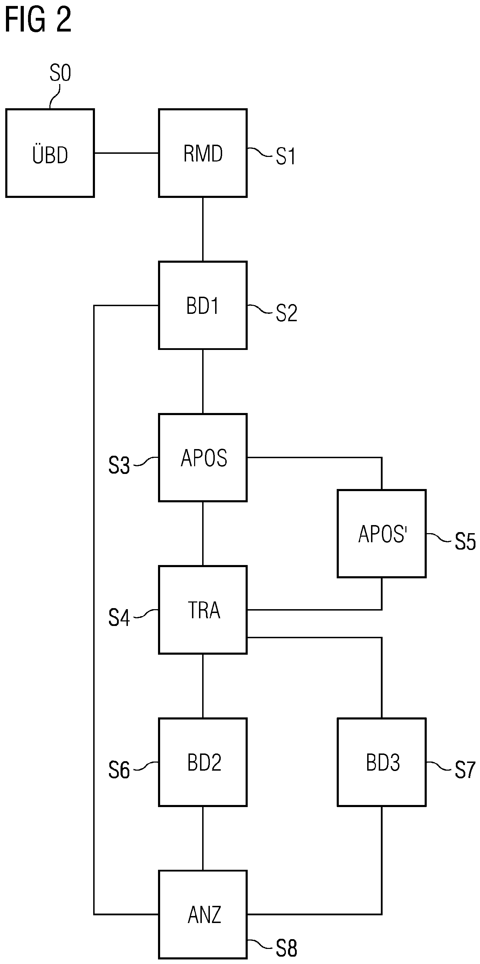

Das Computersystem 12 ist ausgebildet ein, erfindungsgemäßes Verfahren auszuführen, wie es bspw. mit Bezug zu Figur 2 näher beschrieben ist. Das Computersystem 12 steht zu diesem Zweck mit dem Hauptkörper bzw. der Basiseinheit 4 der Mammographie-Anlage 2 zum Datenaustausch in Verbindung. Es können bspw. Steuersignale für die Anlage 2 für die Mammographie-Untersuchung vom Computersystem 12 an die Basiseinheit 4 übertragen werden. Dazu können bspw. verschiedene, jeweils auf eine Untersuchungsart abgestimmte Messprotokolle in einem Speicher 18 hinterlegt sein und durch einen Nutzer, bspw. einen Arzt oder medizinisch-technisch geschultes Personal, vor der Messung entsprechend einer medizinischen Fragestellung ausgewählt werden. Andererseits werden aufgenommene Röntgenprojektionsmessdaten RPM für eine erfindungsgemäße Weiterverarbeitung in dem Computersystem 12 von der Mammographie-Anlage 2 übertragen. Die Verbindung 22 ist in bekannter Weise kabelgebunden oder kabellos über entsprechende Schnittstellen realisiert.

Das Computersystem 12 umfasst eine Vielzahl von Einheiten.

Das Computersystem 12 umfasst eine Erfassungseinheit 20. Die Erfassungseinheit 20 kann als Datenschnittstelle ausgebildet sein und dient dazu, Röntgenprojektionsmessdaten RMD von dem Hauptkörper 4 im Computersystem 12 zu erfassen und ggf. in einem Speicher 18 anzulegen und/oder einer Rekonstruktionseinheit 28 zuzuführen.

Das Computersystem 12 umfasst ferner eine Rekonstruktionseinheit 28. Diese ist ausgebildet, die Röntgenprojektionsmessdaten RPM entsprechend dem ausgewählten Messprotokoll in Bilddatensätze BD1, BD2, BD3 zu überführen. Hierbei können Daten-Vorverarbeitungsschritte, aber auch Artefakt-Korrekturen mit umfasst sein. Die angewendeten Rekonstruktionsvorschriften sind an sich bekannt und bedürfen daher keiner weiteren Beschreibung.

Das Computersystem 12 umfasst ferner eine Bestimmungseinheit 30. Diese ist eingerichtet, eine aktuelle Position APOS einer Läsion L in dem zu untersuchenden Brustgewebe BG zu bestimmen. Die aktuelle Position APOS wird in Bezug auf das Referenz-Koordinatensystem der Mammographie-Anlage 2 bestimmt, welches in dieser Ausführung auch das Referenz-Koordinatensystem des Entnahme-Instruments 17 ist. Die Bestimmungseinheit 30 ist ferner eingerichtet, ggf. eine Segmentierung der Läsion durchzuführen, um die aktuelle Position in den rekonstruierten Bilddaten, hier dem ersten Bilddatensatz BD1 zu bestimmen. Dazu kann die Bestimmungseinheit 30 auch eingerichtet sein, um die aktuelle Position APOS der Läsion L mit einer Referenz-Position in einem Referenz-Bilddatensatz zu registrieren. Der Referenz-Bilddatensatz kann dabei aus einer vorherigen radiologischen Untersuchung desselben Untersuchungsobjektes bezüglich des Brustgewebes BG vorhanden und abrufbar in einem PACS, RIS oder HIS einer medizinischen Einrichtung bzw. dem Speicher 18 der Mammographie-Anlage 2 hinterlegt sein.

Das Computersystem 12 kann ferner optional eine Ermittlungseinheit 26 umfassen. Diese ist eingerichtet, basierend auf der bestimmten, aktuellen Position APOS der Läsion L eine Entnahme-Trajektorie TRA für das Entnahme-Instrument 17 zu ermitteln, die mittels der Biopsieeinheit 15 vollzogen werden kann, um eine Gewebeprobe der Läsion L zu erhalten. Die Entnahme-Trajektorie TRA ist dadurch gekennzeichnet, dass sie die aktuelle Position APOS der Läsion bzw. die Läsion L zumindest einmal schneidet oder trifft. Alternativ kann die Entnahme-Trajektorie manuell durch einen Benutzer bestimmt werden.

Vorliegend sind insbesondere die genannten Einheiten 26, 28 und 30 als getrennte Module innerhalb des Computersystems 12 ausgestaltet, die, wo erforderlich, in Datenaustausch miteinander stehen. Alternativ können alle genannten Einheiten als eine Recheneinheit integriert sein, sei es in Form einer körperlichen oder funktionalen Integrität.

Das Computersystem 12 kann mit einem computerlesbaren Datenträger 24 zusammenwirken, insbesondere um durch ein Computerprogramm mit Programmcode ein erfindungsgemäßes Verfahren durchzuführen. Weiterhin kann das Computerprogramm auf dem maschinenlesbaren Träger 24 abrufbar gespeichert sein. Insbesondere kann es sich bei dem maschinenlesbaren Träger 24 um eine CD, DVD, Blu-Ray Disc, einen Memory-Stick oder eine Festplatte handeln.

Insbesondere die Einheiten 26, 28 und 30 können in Form von Hard- oder in Form von Software ausgebildet sein. Beispielsweise sind die Einheiten als sogenannte FPGAs (Akronym für das englischsprachige "Field Programmable Gate Array") ausgebildet oder umfassen eine arithmetische Logikeinheit.

Auf dem Speicher 18 des Computersystems 12 kann wenigstens ein Computerprogramm gespeichert sein, welches alle Verfahrensschritte des erfindungsgemäßen Verfahrens durchführt, wenn das Computerprogramm auf dem Computer ausgeführt wird. Das Computerprogramm zur Ausführung der Verfahrensschritte des erfindungsgemäßen Verfahrens umfasst Programmcode. Weiterhin kann das Computerprogramm als ausführbare Datei ausgebildet sein und/oder auf einem anderen Rechensystem als dem Computersystem 12 gespeichert sein. Beispielsweise kann die Mammographie-Anlage 2 so ausgelegt sein, dass das Computersystem 12 das Computerprogramm zum Ausführen des erfindungsgemäßen Verfahrens über ein Intranet oder über das Internet in seinen internen Arbeitsspeicher lädt. Alternativ kann vorgesehen sein, dass das Computersystem 12 selbst Teil eines Internets oder Intranets, bspw. eines HIS (Hospital Information System) oder eines RIS (Radiology Information System) ist und Zugriff auf verschiedene Mammographie-Anlagen 2 einer medizinischen Einrichtung hat, um das erfindungsgemäße Verfahren zentral für verschiedene Anlagen 2 auszuführen.

**Figur 2** zeigt eine schematische Darstellung eines erfindungsgemäßen Verfahrens gemäß einem Ausführungsbeispiel der vorliegenden Erfindung. Das Verfahren umfasst eine Vielzahl von Schritten.

In einem ersten Schritt S1 erfolgt ein Erfassen von ersten Röntgenprojektionsmessdaten RMD. Das Erfassen umfasst sowohl ein Erfassen von Röntgenstrahlung mittels eines Röntgenstrahlendetektors 7 als auch ein Erfassen der Röntgenprojektionsmessdaten RMD im Computersystem 12. Die Röntgenprojektionsmessdaten umfassen wenigstens zwei Röntgenprojektionen aus zwei verschiedenen Blickrichtungen. Mit anderen Worten werden in diesem Schritt Röntgenstrahlenquelle 6 und Röntgenstrahlendetektor 7 wenigstens einmal gemeinsam relativ zum dem zu untersuchenden Brustgewebe BG verschwenkt. Kennzeichnend für diesen Schritt ist, das sich in dem zu untersuchenden Brustgewebe ein Kontrastmittel, bevorzugt Jod angereichert hat. Dazu kann das Untersuchungsobjekt zuvor eine Kontrastmittelinjektion erhalten haben. Ferner ist kennzeichnend, dass die Röntgenphotonen, die für die Erzeugung der Röntgenprojektionsmessdaten eingesetzt werden, eine (mittlere) Quantenenergie aufweisen, die oberhalb der Röntgenabsorptionskante des Kontrastmittels liegt. Dies bewirkt einen guten intrinsischen Bildkontrast zwischen Läsion und umliegendem Brustgewebe, sodass allein basierend auf diesen ersten Röntgenprojektionsmessdaten RMD eine aktuelle Position APOS ermittelt werden kann.

Hochenergetische Röntgenstrahlung im Sinne der Erfindung ist als Röntgenstrahlung zu verstehen, die eine mittlere Quantenenergie in Bezug auf das emittierte Energiespektrum aufweist, welche oberhalb der Röntgenabsorptionskante des Kontrastmittels liegt. Um dies zu erreichen, wird die Röntgenstrahlenquelle 6 bevorzugt mit einer Röhrenspannung zwischen 40 und 49 kV, bevorzugt 45 bis 49 kV beaufschlagt.

Optional kann ein Schritt S0 vorgesehen sein. In diesem Schritt erfolgt nach einer Kontrastmittelgabe die Erfassung von Übersichtsprojektionsmessdaten, die zu einem Übersichtsbilddatensatz ÜBD rekonstruiert und bevorzugt mittels Anzeigeeinheit 10 dem Benutzer angezeigt wird. Diese Übersichtsaufnahme weist ein gegenüber dem ersten Bilddatensatz BD1 vergrößertes Bildfeld auf und dient einer groben Lokalisierung der Läsion L in dem Brustgewebe BG. Der Übersichtsbilddatensatz ÜBD deckt bevorzugt die gesamte Brust ab. Anhand der Übersichtsaufnahme ÜBG kann eine Neu-Positionierung des Brustgewebes BG in der Kompressionseinheit 8 oder eine Neupositionierung bzw. Einstellung von Röntgenstrahlenquelle 6, insbesondere umfasster Kollimator und/oder des Röntgenstrahlendetektors 7 erfolgen. Die Übersichtsaufnahme ÜBD kann insbesondere als Tomosynthese-Bilddatensatz ausgebildet sein, der vorteilhaft einen schichtartigen Überblick über das Brustgewebe in drei Dimensionen erlaubt.

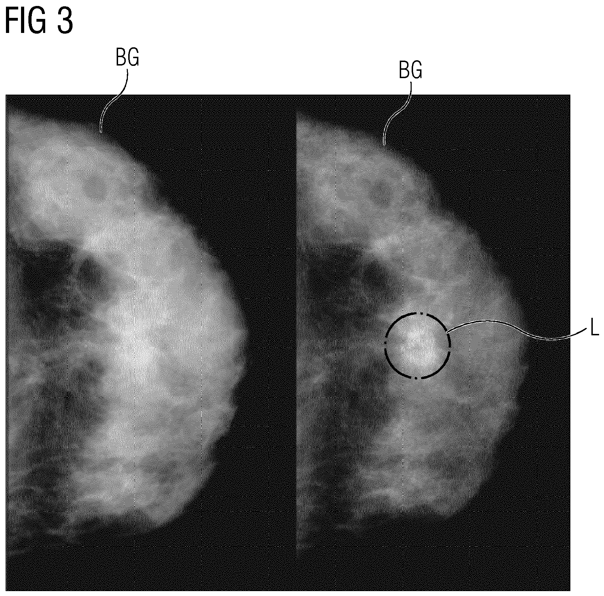

In einem weiteren Schritt S2 erfolgt eine Bildrekonstruktion aus den ersten Projektionsmessdaten RMD auf an sich bekannte Weise. Aus diesen wird ein ersten Bilddatensatz BD1 erstellt, wie er beispielhaft in Figur 3, rechte Seite gezeigt ist. Die Art der Rekonstruktion kann insbesondere auf die Art der ersten Röntgenprojektionsmessdaten RMD angepasst sein. Umfassen diese bspw. eine Vielzahl von Projektionsmessdaten aus einer Vielzahl von verschiedenen Blickrichtungen, werden daraus verschiedene Schichten bestimmten Abstands und insgesamt ein dreidimensionaler Datensatz errechnet.

**Figur 3** zeigt eine Gegenüberstellung von Mammographie-Bilddaten gemäß einer Ausführung des Stands der Technik und einer Ausführungsform der vorliegenden Erfindung. Links erkennt man Brustgewebe BG umfassend eine Läsion L, welches mittels niederenergetischer Röntgenstrahlung unter Verwendung eines Kontrastmittels abgebildet wurde. Die Läsion L ist schlecht erkennbar und noch weniger gut gegenüber dem umgrenzenden Gewebe BG abgrenzbar. Rechts hingegen ist dasselbe Brustgewebe BG unter Verwendung von hochenergetischer Röntgenstrahlung abgebildet worden. Hier ist die Läsion L deutlich heller als das umgebende Brustgewebe BG, sodass eine Positions-/Größen- und/oder Volumenbestimmung offensichtlich deutlich erleichtert ist.

In einem weiteren Schritt S3 erfolgt die Bestimmung der aktuellen Position APOS der Läsion L in dem ersten Bilddatensatz BD1 mit Bezug zum Referenz-Koordinatensystem der Mammographie-Anlage 2, insbesondere wird die Tiefe, aber auch deren Ausdehnung und/oder ihr Volumen bestimmt. Zu diesem Zweck kann eine intensitätsbasierte und/oder einer gradientenbasierte Segmentierung vorgesehen sein, um alle Bildelemente, Pixel oder Voxel zu identifizieren, die zur Läsion L gehören. Die Positionsbestimmung kann alternativ oder zusätzlich auf Nutzer-Eingaben basieren, bspw. händischen Markierung mittels Maus und Cursor in dem dargestellten ersten Bilddatensatz BD1.

In einem weiteren, optionalen Schritt S5 kann eine Registrierung der erkannten, aktuellen Position der Läsion mit einer Referenz-Position der Läsion in einem Referenz-Bilddatensatz, bspw. dem vorhandenen Dualenergie-Subtraktionsbild, einer vorhergehenden radiologischen Diagnosesitzung, erfolgen. Damit wird die Lokalisierung, also die aktuelle Position APOS der Läsion L entweder verbessert oder lediglich bestätigt. Die Registrierung kann unter Erkennung und Registrierung wenigstens einer weiteren anatomischen Landmarke in den beiden zu registrierenden Bilddatensätzen erfolgen, um eine genauere aktuelle Position APOS' der Läsion zu ermitteln. Die anatomischen Landmarken kann bspw. die Hautlinie, die Mamilla, ein Blutgefäßverlauf oder Mikrocalc, sofern vorhanden, sein.

Mittels der aktuellen Position APOS, APOS' kann im Folgenden in einem optionalen Schritt S4 eine Entnahme-Trajektorie TRA und entsprechende Steuersignale für die Entnahme-Einheit 15 bzw. das Entnahme-Instrument 17 ermittelt werden. Dieser Trajektorie folgt das Entnahme-Instrument 17 bei einer anschließenden Gewebeentnahme aus der Läsion L. Die Entnahme-Trajektorie TRA trifft oder kreuzt die aktuelle Position APOS, APOS' wenigstens einmal, bevorzugt mehrmals. Mehrfaches Schneiden der aktuellen Position ist insbesondere dann vorteilhaft, wenn die Läsion vergleichsweise groß ist und Gewebeproben aus verschiedenen Bereichen der Läsion L, bspw. ihrem Zentrum und einem Randbereich genommen werden sollen. Dieser Schritt kann manuell, aber auch automatisch durch die Recheneinheit ausgeführt werden.

Es sei nochmals explizit darauf hingewiesen, dass die Gewebe-Entnahme selbst nicht von der hiesigen Erfindung ausgenommen ist.

In weiteren optionalen Schritten S6 und S7 können jeweils erneut erfindungsgemäß zweite bzw. dritte Röntgenprojektionsmessdaten erfasst und zu Bilddaten BD2 und BD3 rekonstruiert werden. Die Erfassung der Messdaten sowie die Rekonstruktion der Bilddaten BD2 und BD3 entspricht jeweils der Ausgestaltungsvariante, die auch für die ersten Röntgenprojektionsmessdaten bzw. die ersten Bilddaten angewandt wurden.
Die zweiten Bilddaten BD2 werden während einer Bewegung des Entnahme-Instruments 17 entlang der Entnahme-Trajektorie TRA erzeugt. Sie dienen einer Überwachung der Bewegung des Entnahme-Instruments 17 uns ermöglichen einen schnellen manuellen Eingriff durch den Nutzer, bspw. bei unvorhergesehener Patientenbewegung. Bevorzugt werden zu mehreren Zeitpunkten innerhalb der Bewegung des Entnahme-Instruments zweite Bilddaten BD2 erzeugt. Die dritten Bilddaten BD3 werden nach Abschluss der Bewegung des Entnahme-Instruments 17, also nach einer Entnahme einer Gewebe-Probe erzeugt. Sie dienen einer Überprüfung des Brustgewebes auf Verletzungen sowie einer Kontrolle, ob tatsächlich Gewebe der Läsion L an der gewünschten Position entnommen wurde.

Sowohl zweite als auch dritte Bilddatensätze BD2, BD3 werden unter Kontrastmitteleinfluss erzeugt und weisen daher einen vergleichbar guten Bildkontrast in Bezug auf die Läsion L auf wie der erste Bilddatensatz BD1.

Alle hier erläuterten Verarbeitungsschritte erfüllen das Kriterium, dass sie instantan, bzw. quasi-instantan ausgeführt werden. Derart wird eine sofortige Anzeige der Bilddatensätze BD1, BD2, BD3 ohne merkliche Zeitverzögerung ermöglicht.

Insofern ist in einem letzten Schritt S8 eine Anzeige der Bilddatensätze BD1, BD2, BD3 nach Erzeugung vorgesehen, sodass der Nutzer schnell und direkt die Bildinformation analysieren kann.

Im Folgenden wird die Erfindung nochmal kurz zusammengefasst:
Die Erfindung basiert auf der Erkenntnis, dass Läsionen in Brustgewebe, insbesondere Tumoren bereits bei einer Hochenergieaufnahme einen guten Bildkontrast liefern, der für Biopsie-Zwecke ausreichend ist. Eine Hochenergieaufnahme wird mit Röntgenphotonen erzeugt, die eine (mittlere) Quantenenergie oberhalb der Röntgenabsorptionskante eines Kontrastmittels, bevorzugt Jod, aufweisen. Dies bewirkt einen höheren Kontrast als bei einer Standard-Mammografie, die typischerweise mit Röntgenröhrenspannungen zwischen 25kV und 35kV durchgeführt werden. Derart wird vorteilhaft eine reduzierte Strahlendosis für den Patienten erreicht, welche bei einer Hochenergieaufnahme nur etwa 20% bis 50 % einer Standard-Mammografie beträgt. Würde dazu, wie üblich, eine dual-energetische Subtraktionsaufnahme als Übersichtsbilddatensatz verwendet, wäre die Dosis in der Größenordnung von 120% bis 150% der Dosis einer Standard-Mammografie. Gegenüber einer konventionellen stereotaktischen Biopsie-Überwachung beträgt die Dosis nur 20% bis 50%, gegenüber einer vollen dualenergetischen Aufnahme mit gewichteter Subtraktion wäre die Dosiseinsparung sogar erheblich größer. Es ergibt sich weiter ein schnellerer Ablauf der Biopsie und somit eine geringes Maß an Anfälligkeit für Bewegungen des Brustgewebes.

Wo noch nicht explizit geschehen, jedoch sinnvoll und im Sinne der Erfindung, können einzelne Ausführungsbeispiele, einzelne ihrer Teilaspekte oder Merkmale mit einander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Verfahren zur Überwachung einer Gewebeentnahme mittels eines Röntgen-Bildgebungssystems (2), wobei die Gewebe-Entnahme mittels eines Entnahme-Instruments (17) aus einer Läsion (L) in einem zu untersuchenden Brustgewebe (BG) erfolgt, umfassend die Schritte
- Erfassen (S1) von ersten durch ein Kontrastmittel beeinflussten Röntgenprojektionsmessdaten (RMD) des in einem Bildfeld des Röntgen-Bildgebungssystems befindlichen Brustgewebes,
- Rekonstruieren (S2) eines ersten Bilddatensatzes (BD1) auf Basis der erfassten Röntgenprojektionsmessdaten,
- Bestimmen (S3) anhand des rekonstruierten ersten Bilddatensatzes einer aktuellen Position (APOS) der Läsion in Bezug zu einem Referenz-Koordinatensystem,
wobei die kontrastmittelbeeinflussten ersten Röntgenprojektionsmessdaten mit Röntgenphotonen erzeugt werden, die eine Photonenenergie oberhalb einer Absorptionskante des eingesetzten Kontrastmittels aufweisen.

2. Verfahren nach Anspruch 1, welches auch umfasst:
- Ermitteln (S4) einer Entnahme-Trajektorie (TRA) für das Entnahme-Instrument, die die bestimmte aktuelle Position der Läsion umfasst.

3. Verfahren nach Anspruch 1 oder 2, wobei die Röntgenphotonen von einer Röntgenstrahlenquelle (6) des Röntgen-Bildgebungssystems emittiert werden, an welcher eine Spannung anliegt, die in einem Bereich von 40kV bis 49kV liegt.

4. Verfahren nach Anspruch 1, 2 oder 3, wobei die Röntgenphotonen von einer Röntgenstrahlenquelle des Röntgen-Bildgebungssystems emittiert werden, an welcher eine Spannung anliegt, die in einem Bereich von 45kV bis 49kV liegt.

5. Verfahren nach einem der vorhergehenden Ansprüche, wobei der erste Bilddatensatz wenigstens zwei zwei-dimensionale oder einen drei-dimensionalen Bilddatensatz umfasst.

6. Verfahren nach einem der vorhergehenden Ansprüche, wobei vor Erfassen der ersten Röntgenprojektionsmessdaten durch das Kontrastmittel beeinflusste Übersichtsprojektionsmessdaten (ÜBD) erfasst werden.

7. Verfahren nach einem der vorhergehenden Ansprüche 2 bis 6, wobei die aktuelle Position der Läsion in dem ersten Bilddatensatz vor dem Ermitteln der Entnahme-Trajektorie mit einer Referenzposition der Läsion in einem Referenz-Bilddatensatz registriert wird.

8. Verfahren nach Anspruch 7, wobei für die Registrierung die Position wenigstens einer anatomischen Landmarke in dem ersten Bilddatensatz und dem Referenz-Bilddatensatz berücksichtigt wird.

9. Verfahren nach einem der vorhergehenden Ansprüche 2 bis 8, wobei wenigstens zu einem Zeitpunkt zweite durch das Kontrastmittel beeinflusste Röntgenprojektionsmessdaten erfasst und zu einem zweiten Bilddatensatz (BD2) rekonstruiert werden (S6), während das Entnahme-Instrument eine Bewegung entlang der Entnahme-Trajektorie vollzieht.

10. Verfahren nach einem der vorhergehenden Ansprüche 2 bis 9, wobei dritte durch das Kontrastmittel beeinflusste Röntgenprojektionsdaten erfasst und zu einem dritten Bilddatensatz (BD3) rekonstruiert werden (S7), nachdem das Entnahme-Instrument die Bewegung entlang der Entnahme-Trajektorie vollzogen hat.

11. Verfahren nach einem der vorhergehenden Ansprüche 2 bis 10, wobei der erste, der zweite und/oder der dritte Bilddatensatz instantan einem Benutzer angezeigt wird (S8).

12. Recheneinheit (12) zur Überwachung einer Gewebeentnahme mittels eines Röntgen-Bildgebungssystems (2), wobei die Gewebe-Entnahme mittels eines Entnahme-Instruments (17) aus einer Läsion (L) in einem zu untersuchenden Brustgewebe (BG) erfolgt, aufweisend Mittel zum Durchführen eines Verfahrens nach einem der Ansprüche 1 bis 11.

13. Röntgen-Bildgebungssystem (2) zur Überwachung einer Gewebeentnahme, wobei die Gewebe-Entnahme mittels eines Entnahme-Instruments (17) aus einer Läsion (L) in einem zu untersuchenden Brustgewebe (BG) erfolgt, umfassend
- eine Röntgenstrahlenquelle (6) zum Emittieren von Röntgenphotonen,
- eine Erfassungseinheit (7, 20) zum Erfassen von ersten durch ein Kontrastmittel beeinflussten Röntgenprojektionsmessdaten (RMD) des in einem Bildfeld des Röntgen-Bildgebungssystems befindlichen Brustgewebes,
- eine Rekonstruktionseinheit (28) zum Rekonstruieren eines ersten Bilddatensatzes (BD1) auf Basis der erfassten Röntgenprojektionsmessdaten,
- eine Bestimmungseinheit (30) zum Bestimmen einer aktuellen Position (APOS, APOS') der Läsion anhand des rekonstruierten ersten Bilddatensatzes in Bezug zu einem Referenz-Koordinatensystem,
wobei die Röntgenstrahlenquelle ausgebildet ist, Röntgenphotonen zu emittieren, die eine Photonenenergie oberhalb einer Absorptionskante des eingesetzten Kontrastmittels aufweisen, wobei diese Röntgenphotonen zur Erzeugung der ersten Röntgenprojektionsmessdaten eingesetzt werden.

14. Röntgen-Bildgebungssystem nach Anspruch 13, umfassend
- eine Ermittlungseinheit (26) zum Ermitteln einer Entnahme-Trajektorie (TRA) für das Entnahme-Instrument, die die bestimmte Position der Läsion schneidet, und/oder
- eine Anzeige-Einheit (10) zum instantanen Anzeigen (S8) des ersten, zweiten und/oder des dritten Bilddatensatzes (BD1, BD2, BD3) für einen Benutzer.

15. Computerprogramm, welches direkt in eine Speichereinheit (18) des Röntgen-Bildgebungssystems (2) nach Anspruch 13 ladbar ist, mit Programmabschnitten, um die Schritte des Verfahrens nach einem der Ansprüche 1 bis 11 auszuführen, wenn das Computerprogramm in einer Recheneinheit (12) des Röntgen-Bildgebungssystems ausgeführt wird.

16. Computerlesbares Medium (24), auf welchem von einer Recheneinheit (12) des Röntgen-Bildgebungssystems (2) nach Anspruch 13 einlesbare und ausführbare Programmabschnitte gespeichert sind, um alle Schritte des Verfahrens nach einem der Ansprüche 1 bis 11 auszuführen, wenn die Programmabschnitte von der Recheneinheit des Röntgen-Bildgebungssystems ausgeführt werden.
